# EUROPEAN PATENT SPECIFICATION

(11) **EP 1 711 468 B1**
(45) Date of publication and mention of the grant of the patent: **14.05.2008**
(21) Application number: 05715236.5
(22) Date of filing: 01.02.2005
(51) Int. Cl.: C07D 215/56, A61K 31/4709, A61P 31/04

(54) **PROCESS FOR PREPARING PURIFIED CIPROFLOXACIN**
VERFAHREN ZUR HERSTELLUNG VON AUFGEREINIGTEM CIPROFLOXACIN
PROCEDE DE PREPARATION DE CIPROFLOXACINE PURIFIEE

(30) Priority: 02.02.2004 DE 102004005186
(43) Date of publication of application: 18.10.2006
(73) Proprietor: KRKA, tovarna zdravil, d.d., Novo mesto, 8501 Novo mesto (SI)
(72) Inventor: RUZIC, Milos, 3000 Celje (SI); PUCELJ, Joze, 8000 Novo mesto (SI); TOMSIC, Zdenka, 8000 Novo mesto (SI); MAKUC, Simon, 8000 Novo mesto (SI); BRNE, Peter, 6250 Ilirska Bistrica (SI); BARUT, Milos, 1000 Ljubljana (SI); STRANCAR, Ales, 5270 Ajdovscina (SI)
(74) Representative: UEXKÜLL & STOLBERG
(86) International application number: PCT/EP2005/000975
(87) International publication number: WO 2005/075430

(56) References cited:
- EP-A- 0 287 926
- CA-C- 1 326 239
- US-A- 5 914 401

## Description

### FIELD OF THE INVENTION

The present invention relates to a process for preparing purified ciprofloxacin which substantially reduces undesired impurities and therefore makes the obtained purified ciprofloxacin particularly suitable for inclusion into infusion and injection solutions.

### BACKGROUND OF THE INVENTION

Ciprofloxacin belongs to the group of quinolone carboxylic acids which are known to be effective antibacterial agents. It is known that in particular impurities of these substances can cause vein irritation upon infusion and injection of corresponding solutions.

Moreover, it has also been observed with these active ingredients that solutions of them for parenteral use often suffer from the formation of undesired precipitates which may have been caused by the presence of impurities.

There have been several attempts to avoid the formation of precipitates and to reduce the level of impurities by specific processes in case of ciprofloxacin.

EP-A-138 018 discloses lactate solutions of ciprofloxacin which also contain another acid to prevent the formation of precipitates. The acids are selected from lactic acid, methanesulfonic acid, propionic acid or succinic acid. The content of lactic acid in the prepared infusion solutions can be from 0,1 and as high as 90%. However, it has been shown that ciprofloxacin solutions having a lactic acid content between 0,1 to 90% are physiologically not well tolerated by the patient.

According to EP-A-0 219 784 infusion solutions of ciprofloxacin are prepared which also contain one or more physiologically tolerated acid, chosen from the group of hydrochloric, methanesulphonic, propionic, succinic, glutaric, citric, fumaric, maleinic, tartric, glutaminic, glucoronic, galacturonic, ascorbinic, phosphoric, adipinic, hydroxyacetic, sulphuric, nitric, acetic, malic, L-asparaginic and lactic acid. While these solution have a diminished toxicity, they still have an undesirably low stability.

In the attempt to improve stability of infusion solutions while using lower concentrations of added acid, EP-A-287 926 discloses solutions of ciprofloxacin for parenteral application wherein ciprofloxacin of a high purity is used.

The solutions comprise only 1 to 10 ppm of impurities which tend to precipitate after long-term storage. The purification of ciprofloxacin is effected by dissolving it in acidic media at a temperature ranging from ambient temperature to boiling temperature, optionally with addition of activated carbon, and leaving the solution to stand for 0,15 to 150 hours. The solution is then filtered and the ciprofloxacin is precipitated by adding basic reagents. In this way a reduction of precipitation from solutions was achieved.

According to WO 99/02160 the problem of formation of precipitates can be overcome by the use of glass vessels with a special inner coating made of silicone.

WO 02/26233 relates to stable infusion solutions of ciprofloxacin which can be obtained by mixing the active ingredient in an aqueous solution with sulphuric acid or sodium hydrogen sulphate in a quantity equal to or less than 0.96 mole per mole of active ingredient. This allows the reduction of the acid content and enables a larger proportion of impurities to be tolerated by the active ingredient.

WO 01/78731 discloses infusion solutions of ciprofloxacin which contain one or more physiologically acceptable acid, which is a mono or diester of orthophosphoric acid.

WO 01/78732 relates to infusion solutions of ciprofloxacin with improved storage stability containing sulphuric acid which allows to reduce the acid content of the solution.

Further, the HPLC separation of quinolones in acidic media is described by O. Ballesteros et al. in Chromatographia, 2002, 56, 413-421.

However, the above mentioned processes are still suffering from the disadvantages that they are time consuming and costly and that the addition of a further acid may still lead to vein irritations after parenteral application of the ciprofloxacin solutions. Moreover, these prior art processes often do not lead to ciprofloxacin having a purity which is satisfactory for the preparation of infusion and injection solutions.

Consequently, there is still a need for an improved process to prepare purified ciprofloxacin avoiding the aforementioned drawbacks and which results to ciprofloxacin which has a purity making it very well suitable for the preparation of parenteral solutions.

This problem is surprisingly solved by the process for preparing purified ciprofloxacin according to claims 1 to 12.

### DETAILED DESCRIPTION OF THE INVENTION

The process according.to the invention for preparing purified ciprofloxacin comprises
(a) contacting a solution of ciprofloxacin having a pH value of 11 or more with a solid phase which is stable at the pH of the solution, to give a solution of purified ciprofloxacin,
(b) optionally recovering purified ciprofloxacin, and
(c) optionally converting the purified ciprofloxacin to a salt thereof
and (i) wherein step (a) is effected in a chromatographic column with the solid phase acting at least as part of the stationary phase of the column;
and/or (ii) wherein the solid phase comprises a polymer.

It has surprisingly been shown that this process is able to result in ciprofloxacin wherein impurities, which have a tendency to precipitate from solutions, in particular after storage of solution, are reduced to levels below 10 ppm by weight.

Examples of such impurities which can be reduced to the desired level by the process according to the invention are mentioned in EP-A-287 926 and referred to as "poly condensation products" having the following general formula

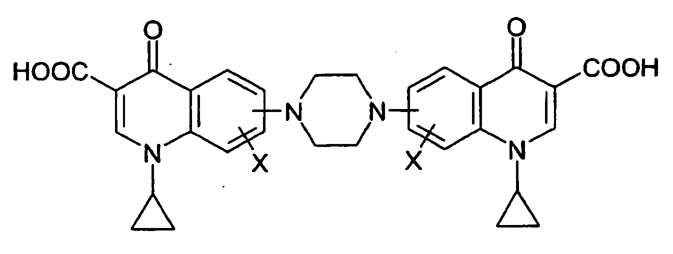

The process according to the invention also allows to reduce the content of the following impurities to the desired level: 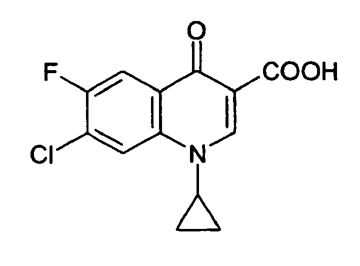 or

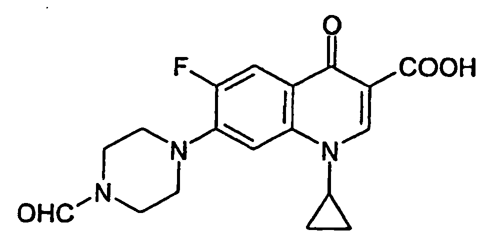

It has unexpectedly been found that the contracting of ciprofloxacin solution with the solid phase at a pH value of more than 11 in step (a) of the process results in the reduction of a number of impurities to a level which allows the preparation of parenteral solutions not suffering from stability problems in terms of formation of precipitates upon storage. It was also found out that this process allows the purification of solutions having a high concentration of ciprofloxacin which makes the process also from an economic standpoint very attractive. In contrast to this, prior art processes for chromatographic purification of ciprofloxacin use acidic conditions which only allow use of solutions to be purified which have a lower concentration of ciprofloxacin.
It has also been shown that in the process according to the invention the pH value of the solution of ciprofloxacin is preferably in the range of 11 to 14 and more preferably in the range of 11.5 to 13.

In a preferred embodiment of the process according to the invention, step (a) is effected:
- by using a solid phase extraction (SPE) method in a batch mode by mixing a solution of ciprofloxacin having a pH value of 11 or more with a solid phase which comprises a polymer and which is stable at the pH of the solution in an appropriate vessel or
- by using a solid phase extraction (SPE) method in a chromatographic column mode with the solid phase acting at least as part of the stationary phase of the column. It is even more preferred that the solid phase is the stationary phase of the column. In these preferred embodiments, step (a) can be regarded as a chromatographic/SPE purification.

As pointed out above the solid phase used in step (a) is stable at the pH of the solution of ciprofloxacin and in the preffered embodiment comprises a polymer. This polymer is preferably hydrophobic. Particularly preferred polymers are those based on poly(styrene-co-divinylbenzene) polymers and their derivatives or poly(glycidylmethacrylate-co-ethylendimethacrylate) polymers and their derivatives. These polymers are preferably functionalized with hydrophobic groups and are also preferably porous. Normally the polymers are in the form of particles, membranes or monoliths.

The process according to the invention allows use of ciprofloxacin solutions to be purified which comprise as low as 0.01 to about 90 mg ciprofloxacin/ml solution which corresponds to a saturated solution. However, when not only taking into consideration a high purity, but also economic factors, then it is preferred that the ciprofloxacin solution comprises 10 to 70 and preferably 40 to 50 mg ciprofloxacin/ml solution.

The ciprofloxacin solution comprises an alkaline reacting substance. This is preferably selected from aqueous ammonia or aqueous NaOH or KOH or an aqueous buffer of high pH. Further, it is preferred that the ciprofloxacin solution comprises a polar organic solvent, which is preferably acetonitrile.

Particularly preferred is use of a mixture of aqueous NaOH and acetonitrile.

If step (a) is carried out as a liquid chromatography, then there could as eluent be used also a gradient of alkaline reacting substance and organic solvent.

The ciprofloxacin used in the solution in step (a) can be from various sources. These sources may be the known processes for the preparation of ciprofloxacin which normally use substituted piperazines, for example EP 49355, EP 78362, and EP 176846. A preferred procedure uses a piperazine wherein the secondary amino group has been protected by an ethoxycarbonyl group as it leads to ciprofloxacin having a high purity. Such a procedure is disclosed in :
DD 283 386, EP 0 131 839 B1, EP 0 350 950, ES 2 006 099 and ES 2 015 652.

Therefore, in the process according to the invention it is preferred to use in step (a) ciprofloxacin which has been produced according to the process given in the reaction scheme below. It is even more preferred if the reaction from compound **3** to ciprofloxacin **4** as well as the recovery of the ciprofloxacin **4** is conducted in media having a pH value of > 7.

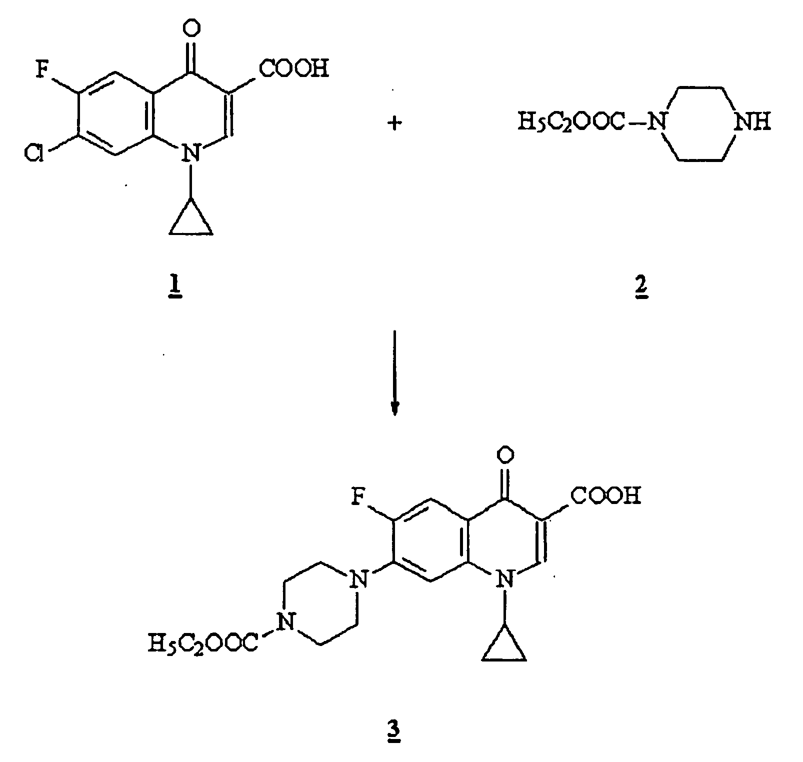

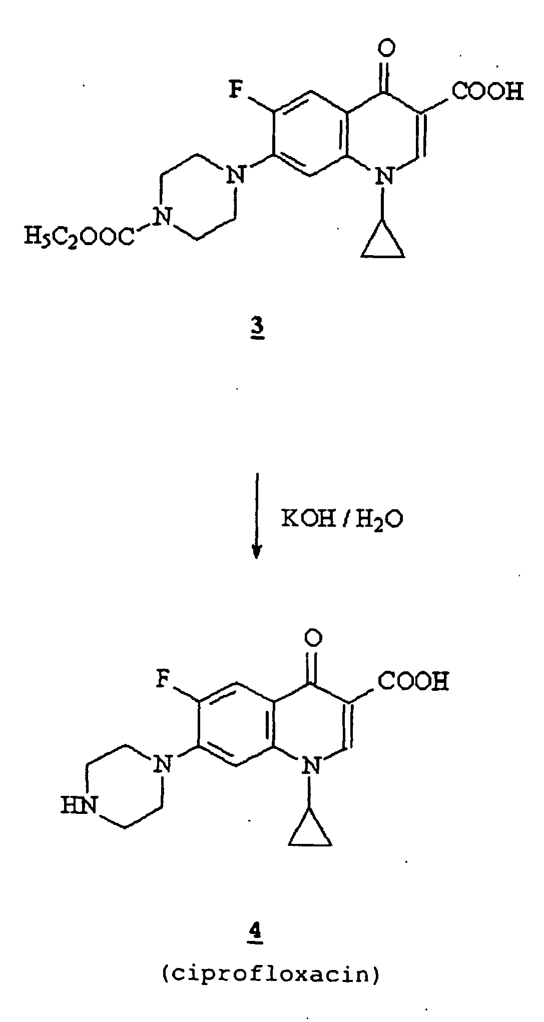

In step (b) of the process according to the invention the purified ciprofloxacin is optionally recovered in a conventional manner (precipitation at pH = 7.4-7.5) which for example could include evaporation of any solvents and drying of ciprofloxacin.

Finally, in step (c) the obtained purified ciprofloxacin can optionally be converted to a salt thereof. Corresponding procedures are well-known in the art and are useful.

The process according to the invention is able to reduce the amount of impurities to a level below 10ppm, preferably below 5ppm. Despite the achievement of such a high purity, the process is also economical as it allows preparation of purified ciprofloxacin in a yield of more than 70 and up to 95%, based on crude ciprofloxacin.

A further advantage of the process according to the invention is the fact that the particularly preferred polymers, which can be used for the contacting of the ciprofloxacin solution in step (a), can be used many times using simple regeneration procedures between the SPE processes. This represents a substantial benefit over a chromatographic purification of ciprofloxacin in acidic media.

The invention is further illustrated in the following examples

### Examples:

### Example 1: Synthesis of 1-cyclopropyl-7-((4-ethoxycarbonyl)-1-piperazinyl)-6-fluoro-1,4-dihydro-4-oxo-quinoline-3-carboxylic acid (3)

A mixture of 110 ml of *N*-methy-2-pyrrolidone, 49.2 g of *N-*ethoxycarbonylpiperazine (**2**) and 35.0 g of 7-chloro-1-cyclopropyl-6-fluoro-1,4-dihydro-4-oxo-quinoline-3-carboxylic (**1**) acid is heated to 130 °C for 9 hours and then cooled to 75 °C. Then 110 ml of ethanol is added and the obtained suspension is allowed to cool to 25 - 30 °C. The suspension is immediately filtered and washed with 100 ml of ethanol. About 41 g of wet title product is produced which can be used for the further synthesis without drying.

### Example 2: Synthesis of crude 1-cyclopropyl-6-fluoro-1,4-dihydro-4-oxo-7-(piperazin-1-yl)quinoline-3-carboxylic acid (4) (ciprofloxacin)

Wet 1-cyclopropyl-7-((4-ethoxycarbonyl)-1-piperazinyl)-6-fluoro-1,4-dihydro-4-oxo-quinoline-3-carboxylic acid from example 1 is suspended in a mixture of 280 ml of water and 38.1 g KOH and heated to reflux. Remaining ethanol should previously be removed by distillation. The solution is refluxed for 3.5 hours, cooled to room temperature and the pH is adjusted to 7.7 - 7.9 with 15 % aqueous HC1. The obtained white suspension is agitated for 30 minutes, the solid separated by filtration and washed with 100 ml of water. About 46 g of wet crude ciprofloxacin are isolated, which can be used for the purification process without drying.

### Example 3: On column purification of ciprofloxacin with Amberchrom as stationary phase

Composition of the system: Pump for liquid chromatography, liquid chromatography column: XK 26/20 (Amersham Biosciences)

Stationary phase: Amberchrom CG-161S, a porous poly(styrene-co-divinylbenzene) polymer, (Tosoh Bioscience), amount of stationary phase: 60 mL (measured in 20% EtOH) Linear velocity of the mobile phase or sample application: 125 cm/h.

### Sample preparation:

Wet crude ciprofloxacin of example 2 is weighted in an amount so that the calculated amount of dry substance is 39 g. The sample is then dissolved in a mixture of 0,2 M NaOH (850 mL) and acetonitrile (17,3 mL). In this way a solution was prepared which comprises 40g ciprofloxacin/L with a pH of about 13 and it was subsequently filtered through a 0,45 µm filter.

### Purification Procedure:

1.) The column is packed with the stationary phase and washed and thus conditioned with at least a 6-fold volume of a mixture of 0,1 M NaOH:acetonitrile (98:2 Vol/Vol) having pH 13.
2.) The prepared sample is flushed over the column and the purity of the eluted fractions obtained is followed with in-process analysis. Fractions are collected as long as they do not contain the impurities which have to be removed.
3.) After finalizing step (2) the column is regenerated by flushing it with a 5-fold volume of a mixture of 0.1 M NaOH : acetonitrile, (30:70 (Vol/Vol) solution. After regeneration the column is washed and thus conditioned with at least a 6-fold volume of a mixture of 0,1 M NaOH:acetonitrile (98:2 Vol/Vol) having pH 13 and after this ready for next purification step.

From the collected fractions acetonitrile is evaporated. The obtained mixture is optionally diluted with purified water and optionally treated for some time with activated charcoal and EDTA for complexation of cations at higher temperatures and filtered. The pH of the remaining alkaline solution is adjusted to 7.4 - 7.5. The obtained white suspension is mixed for about 30 minutes and the white product is filtered off and washed with water. The wet product is very pure ciprofloxacin which can be administered parenterally. The concentration of the impurities N-(1-cyclopropyl-6-fluoro-1,4-dihydro-3-carboxy-4-oxo-7-quinolonyl)-N'-(1-cyclopropyl-1,4-dihydro-3-carboxy-7-chloro-4-oxo-6-quinolonyl)piperazine and N-(1-cyclopropyl-6-fluoro-1;4-dihydro-3-carboxy-4-oxo-7-quinolonyl)-N'-(1-cyclopropyl-6-fluoro-1,4-dihydro-3-carboxy-4-oxo-7- quinolonyl)piperazine is less than 1 ppm, and the concentration of 7-chloro-1-cyclopropyl-6-fluoro-1,4-dihydro-4-oxo-quinoline-3-carboxylic acid is less than 5 ppm.

### Example 4: Synthesis of (3) on a large scale

A mixture of 16.4 1 of *N*-methy-2-pyrrolidone, 6.8 1 of *N-*ethoxycarbonylpiperazine (**2**) and 5.24 kg of 7-chloro-1-cyclopropyl-6-fluoro-1,4-dihydro-4-oxo-quinoline-3-carboxylic acid (**1**) is heated to 125 - 135 °C for 8 - 12 hours , and then cooled to 75 °C. At this temperature 16 1 of ethanol is added and the obtained suspension is allowed to cool to 25 - 30°C. The suspension is immediately centrifuged and washed with 15 1 of ethanol. About 6,5 kg of wet intermediate is produced and the synthesis is allowed to continue without drying the product.

### Example 5: Synthesis of crude (4) (ciprofloxacin) on a large scale

Wet 1-cyclopropyl-7-((4-ethoxycarbonyl)-1-piperazinyl)-6-fluoro-1,4-dihydro-4-oxo-quinoline-3-carboxylic acid (**3**) from example 4 is suspended in a mixture of 42 1 of water and 5.7 kg KOH and heated to reflux. Remaining ethanol should previously be removed by distillation. The solution is refluxed for 3 - 6 hours, cooled to room temperature and the pH is adjusted to 7.7 - 7.9 with 15 % aqueous HC1. The obtained white suspension is agitated for 30 minutes, centrifuged, and washed with 15 1 of water. 13,6 kg of wet crude ciprofloxacin is obtained which can be used for the purification without drying.

### Example 6: Synthesis of crude ciprofloxacin (4)

240 ml of 2-methoxyethanol, 48.9 kg of piperazine anhydrous and 40 kg of 1-cyclopropyl-7-chloro-6-fluoro-1,4-dihydro-4-oxo-3-quinolinecarboxylic acid are charged into a flask. The reaction mixture is refluxed at 127 to 129 °C for 7.5 to 8 hours. The solvent is evaporated under reduced pressure (200 to 250 mbar and at a temperature of 100 to 110 °C) resulting in 170 to 180 ml of 2-methoxyethanol. To the residue 200 ml water are added and the suspension is refluxed at a temperature of 98 to 100 °C for 30 minutes. The reaction mixture is cooled to a temperature of 15 to 18 °C within 30 minutes and it is agitated at this temperature for another 30 minutes. The obtained precipitate is filtered off and washed with 200 ml water which is cooled to 10 to 15 °C.

### Example 7: Purification of the crude ciprofloxacin (4)

The crude ciprofloxacin from example 6 is dissolved in 680 ml aqueous ammonia having a pH of > 12.5, treated with activated charcoal at a temperature between ambient temperature and boiling temperature, filtered and ciprofloxacin is precipitated at a pH of 7.7 - 7.9 at a temperature of 30 - 35 °C. After this pre-purification step, the obtained product is then purified as given in example 3.

### Example 8: On column purification of crude ciprofloxacin using as stationary phase Amberchrom CG161s

Composition of the system: HPLC pump, chromatographic column UV detector
Chromatographic column: XK 16/20 (Amersham Biosciences)
Stationary phase: Amberchrom CG161s, a porous poly(styrene-co-divinylbenzene) polymer, (Tosoh Bioscience); the amount of stationary phase in the column is 4 ml (measured in EtOH:H₂O (20:80) (Vol/Vol)).

Linear velocity of equilibration buffer, sample, regeneration buffer: approximately 30 cm/h in each case.

### Sample preparation:

Wet crude ciprofloxacin is weighed in an amount so that the calculated amount of dry substance is 2.00 g. To this sample 0.55M aqueous ammonia (200 ml) and acetonitrile (4 ml) are added. Afterwards 25% aqueous ammonia (about 4 ml) is added to the sample until a pH value of about 11.5 was reached. In this way a solution having a concentration of about 9g ciprofloxacin/L (1.8M NH₄OH:acetonitrile (98:2 Vol/Vol) is reached.
The sample is subsequently filtered through a 0.45 µm filter.

### Purification Procedure:

1.) The column packed with the stationary phase is washed and thus equilibrated by pumping at least 8 Column Volumes (CV) of 0.55M aqueous ammonia: acetonitrile (98:2 (Vol/Vol) (equilibration buffer) through the column using a linear velocity as given above.
2.) Subsequently the sample is pumped through the column using a linear velocity as given above. Fractions of the eluates are taken and the purity of each fraction is analyzed by a reverse-phase HPLC method. The process is stopped once the concentration of the impurities starts to increase considerably.
3.) After the purification process is completed, the column is regenerated by pumping at least 6 CV of 0.55M NH₄OH:acetonitrile (10:90 (Vol/Vol) (regeneration buffer) through the column using a linear velocity as given above.

### Example 9: On column purification of crude ciprofloxacin using as stationary phase PLRP-S

Composition of the system: HPLC pump, chromatographic column, UV detector
Chromatographic column: XK 16/20 (Amersham Biosciences)
Stationary phase: PLRP-S, a porous poly(styrene-co-divinylbenzene) polymer, (Polymer Laboratories); the amount of stationary phase in the column is 4 ml (measured in EtOH:H₂O (20:80 Vol/Vol).

Linear velocity of equilibration buffer, sample, regeneration buffer: about 30 cm/h in each case.

The sample preparation and the purification procedure were as described in example 8.

### Example 10: On column purification of crude ciprofloxacin using as stationary phase SOURCE 15RPC

Composition of the system: HPLC pump, chromatographic column, UV.detector
Chromatographic column: XK 16/20 (Amersham Biosciences)
Stationary phase: SOURCE 15RPC, a porous poly(styrene-co-divinylbenzene) polymer, (Amersham Biosciences); the amount of stationary phase in the column is 4 ml (measured in EtOH:H₂O (20:80 Vol/Vol).

Linear velocity of equilibration buffer, sample, regeneration buffer: about 30 cm/h in each case.

The sample preparation and the purification procedure were as described in example 8.

### Example 11: Batch purification of crude ciprofloxacin using Amberchrom CG161s

Composition of the system: Beaker, Magnetic mixer
Solid phase: Amberchrom CG161s, a porous poly(styrene-co-divinylbenezene) polymer, (Tosoh Bioscience); the amount of stationary phase is 25 ml (measured in EtOH:H₂O (20:80) (Vol/Vol)).

### Sample preparation:

Wet crude ciprofloxacin base is weighed in an amount so that the calculated amount of dry sample is 9.0 g. To the sample 0.2 M NaOH (196 ml) and acetonitrile (4 ml) are added to adjust pH value of the sample to about 13.0. The concentration of the sample is about 45.0 g/L (0,2 M NaOH:acetonitrile (98:2) (Vol/Vol)).
The sample is subsequently filtered over a 0,45 µm filter.

### Procedure:

1.)The solid phase is equilibrated by mixing 3 CV of 0.1 M NaOH:acetonitrile (98:2) (Vol/Vol) (equilibration buffer) for at least 10 minutes. The mixture is then allowed to settle for at least 5 hours. Subsequently, the equilibration buffer is decanted, leaving the solid phase in the beaker.
2.) Then the sample is added to the equilibrated solid phase and mixed for about 10 minutes. The mixture is allowed to settle for at least 5 hours. Then the sample is decanted and prepared, if necessary, for second Solid Phase Extraction (SPE) step, leaving the solid phase in the beaker. The purity of decanted sample is analyzed by a reversed-phase HPLC method.
3.) The solid phase is regenerated and equilibrated as noted under 1.
4.) If needed the sample is further purified as described under 2.
The procedure 1. and 2. is repeated until a satisfactory purity of the product is reached. After this the ciprofloxacin is isolated by precipitation and the regenerated solid phase can be used for a new batch.

The following examples 12 and 13 illustrate the use of ciprofloxacin purified according to any one of examples 3 and 7 to 11 for the preparation of infusion solutions.

### Example 12: Ciprofloxacin solution for infusion

| | |
|---|---|
| Ciprofloxacin | 200.00 mg |
| Sodium Lactate | 134.00 mg |
| Sodium Chloride | 850.00 mg |
| Hydrochloric Acid | q.s. to a pH= 4.0-4.2 |
| Water for Injection | q.s. to 100.00 ml |

| | |
|---|---|
| "q.s." - as needed | |

First, sodium chloride and then sodium lactate as well as 10% hydrochloric acid solution were added to water. To the obtained solution ciprofloxacin was addded and the pH value of the solution was adjusted with 10% hydrochloric acid solution. Water for injection was added to the give the desired volume of the batch. Subsequently, the solution was filtered by sterile filtration and filled in vials (or plastic bags). These were fitted with rubber closures coated with fluorocarbon film. The filled bottles were finally sterilized in an autoclave.

### Example 13: Ciprofloxacin solution for infusion

| | |
|---|---|
| Ciprofloxacin | 100.00 mg |
| Lactic Acid | 48.50 mg |
| Disodium Edetate | 1.00 mg |
| Hydrochloric Acid | q.s. to a pH= 3.4-3.6 |
| Water for Injection | q.s. to 10.00 ml |

First, disodium edetate and then ciprofloxacin as well as lactic acid were added to water. To the obtained solution 10% hydrochloric acid was added and the pH value was adjusted. Water for injection was added to give the desired volume of the batch. Subsequently, the solution was filtered by sterile filtration and filled in ampoules. The filled ampoules were then sterilized in an autoclave. Prior to use, the obtained solution is diluted with a compatible infusion solution.

## Claims

1. Process for preparing purified ciprofloxacin which comprises
(a) contacting a solution of ciprofloxacin having a pH value of 11 or more with a solid phase which is stable at the pH of the solution, to give a solution of purified ciprofloxacin,
(b) optionally recovering purified ciprofloxacin, and
(c) optionally converting the purified ciprofloxacin to a salt thereof
and (i) wherein step (a) is effected in a chromatographic column with the solid phase acting at least as part of the stationary phase of the column and/or (ii) wherein the solid phase comprises a polymer.

2. Process according to claim 1, wherein the pH of the solution of ciprofloxacin is in the range of 11 to 14.

3. Process according to claim 1 or 2, wherein the pH of the solution of ciprofloxacin is in the range of 11.5 to 13.

4. Process according to any one of claims 1 to 3, when step (a) is effected by mixing the solution of ciprofloxacin with the solid phase in an appropriate vessel.

5. Process according to any one of claims 1 to 3, wherein the solid phase is the stationary phase of the column.

6. Process according to any one of claims 1 to 5, wherein the ciprofloxacin solution comprises aqueous ammonia or aqueous NaOH, KOH or an aqueous buffer of high pH.

7. Process according to any one of claims 1 to 6, wherein the ciprofloxacin solution comprises polar organic solvent.

8. Process according to claim 7, wherein the polar organic solvent comprises acetonitrile.

9. Process according to any one of claims 1 to 8, wherein the polymer is hydrophobic.

10. Process according to any one of claims 1 to 9, wherein the polymer is based on poly(styrene-co-divinylbenzene) polymer or a derivative thereof or poly(glycidylmethacrylate-co-ethylendimethacrylate) polymer or a derivative thereof.

11. Process according to any one of claims 1 to 10, wherein the solution of ciprofloxacin comprises 10 to 70 mg ciprofloxacin/ml solution.

12. Process according to claim 11, wherein the solution of ciprofloxacin comprises 40 to 50 mg ciprofloxacin/ml solution.

## Patentansprüche

1. Verfahren zur Herstellung von gereinigtem Ciprofloxacin, bei dem
(a) eine Lösung von Ciprofloxacin mit einem pH-Wert von 11 oder mehr mit einer festen Phase, die bei dem pH-Wert der Lösung stabil ist, in Kontakt gebracht wird, um eine Lösung von gereinigtem Ciprofloxacin zu ergeben,
(b) gegebenenfalls gereinigtes Ciprofloxacin gewonnen wird und
(c) gegebenenfalls das gereinigte Ciprofloxacin in ein Salz davon umgewandelt wird,
(i) wobei Stufe (a) in einer chromatographischen Säule ausgeführt wird, wobei die feste Phase mindestens als Teil der stationären Phase der Säule wirkt, und/oder
(ii) wobei die feste Phase ein Polymer aufweist.

2. Verfahren nach Anspruch 1, bei dem der pH der Lösung von Ciprofloxacin im Bereich von 11 bis 14 liegt.

3. Verfahren nach Anspruch 1 oder 2, bei dem der pH der Lösung von Ciprofloxacin im Bereich von 11,5 bis 13 liegt.

4. Verfahren nach einem der Ansprüche 1 bis 3, bei dem Stufe (a) ausgeführt wird, indem die Lösung von Ciprofloxacin in einem geeigneten Gefäß mit der festen Phase gemischt wird.

5. Verfahren nach einem der Ansprüche 1 bis 3, bei dem die feste Phase die stationäre Phase der Säule ist.

6. Verfahren nach einem der Ansprüche 1 bis 5, bei dem die Ciprofloxacinlösung wässriges Ammoniak oder wässrige NaOH, KOH oder einen wässrigen Puffer mit hohem pH-Wert enthält.

7. Verfahren nach einem der Ansprüche 1 bis 6, bei dem die Ciprofloxacinlösung polares organisches Lösungsmittel enthält.

8. Verfahren nach Anspruch 7, bei dem das polare organische Lösungsmittel Acetonitril enthält.

9. Verfahren nach einem der Ansprüche 1 bis 8, bei dem das Polymer hydrophob ist.

10. Verfahren nach einem der Ansprüche 1 bis 9, bei dem das Polymer auf Poly(styrol-co-divinylbenzol)polymer oder einem Derivat davon oder Poly(glycidylmethacrylat-co-ethylendimethacrylat)polymer oder einem Derivat davon basiert.

11. Verfahren nach einem der Ansprüche 1 bis 10, bei dem die Lösung von Ciprofloxacin 10 bis 70 mg Ciprofloxacin/ml Lösung enthält.

12. Verfahren nach Anspruch 11, bei dem die Lösung von Ciprofloxacin 40 bis 50 mg Ciprofloxacin/ml Lösung enthält.

## Revendications

1. Procédé de préparation de ciprofloxacine purifiée qui comprend
(a) la mise en contact d'une solution de ciprofloxacine ayant une valeur de pH de 11 ou plus avec une phase solide qui est stable au pH de la solution, pour donner une solution de ciprofloxacine purifiée,
(b) récupérer éventuellement la ciprofloxacine purifiée, et
(c) convertir éventuellement la ciprofloxacine purifiée en l'un de ses sels; et
(i) dans lequel l'étape (a) est réalisée dans une colonne de chromatographie avec la phase solide agissant au moins en tant que partie de la phase stationnaire de la colonne et/ou
(ii) dans lequel la phase solide comprend un polymère.

2. Procédé selon la revendication 1, dans lequel le pH de la solution de ciprofloxacine est dans le domaine de 11 à 14.

3. Procédé selon la revendication 1 ou 2, dans lequel le pH de la solution de ciprofloxacine est dans le domaine de 11,5 à 13.

4. Procédé selon l'une quelconque des revendications 1 à 3, quand l'étape (a) est réalisée en mélangeant la solution de ciprofloxacine avec la phase solide dans un récipient approprié.

5. Procédé selon l'une quelconque des revendications 1 à 3, dans lequel la phase solide est la phase stationnaire de la colonne.

6. Procédé selon l'une quelconque des revendications 1 à 5, dans lequel la solution de ciprofloxacine comprend de l'ammoniaque aqueuse ou NaOH, KOH aqueux ou un tampon aqueux de pH élevé.

7. Procédé selon l'une quelconque des revendications 1 à 6, dans lequel la solution de ciprofloxacine comprend un solvant organique polaire.

8. Procédé selon la revendication 7, dans lequel le solvant organique polaire comprend de l'acétonitrile.

9. Procédé selon l'une quelconque des revendications 1 à 8, dans lequel le polymère est hydrophobe.

10. Procédé selon l'une quelconque des revendications 1 à 9, dans lequel le polymère est à base de polymère de poly(styrène-co-divinylbenzène) ou un dérivé de celui-ci ou un polymère de poly(glycidyl-méthyarylate-co-éthylèndiméthyarylate) ou un dérivé de celui-ci.

11. Procédé selon l'une quelconque des revendications 1 à 10, dans lequel la solution de ciprofloxacine comprend de 10 à 70 mg de ciprofloxacine/ml de solution.

12. Procédé selon la revendication 11, dans lequel la solution de ciprofloxacine comprend de 40 à 50 mg de ciprofloxacine/ml de solution.
